# EUROPÄISCHE PATENTSCHRIFT

(11) **EP 3 116 562 B1**
(45) Veröffentlichungstag und Bekanntmachung des Hinweises auf die Patenterteilung: **04.09.2019**
(21) Anmeldenummer: 15709502.7
(22) Anmeldetag: 13.03.2015
(51) Int. Cl.: A61M 1/16, A61M 39/22, F16K 7/12

(54) **BLUTBEHANDLUNGSKASSETTE MIT FOLIENVENTIL UND ELASTISCHEM ABSTANDHALTER SOWIE BLUTBEHANDLUNGSVORRICHTUNG**
BLOOD TREATMENT CASSETTE HAVING A FILM VALVE AND AN ELASTIC SPACER, AND BLOOD TREATMENT DEVICE
CASSETTE DE TRAITEMENT SANGUIN DOTÉE D'UNE VALVE À FEUILLE ET D'UN ÉLÉMENT D'ESPACEMENT ÉLASTIQUE ET DISPOSITIF DE TRAITEMENT SANGUIN

(30) Priorität: 14.03.2014 DE 102014103490
(43) Veröffentlichungstag der Anmeldung: 18.01.2017
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2015/055281
(87) Internationale Veröffentlichungsnummer: WO 2015/136068

(56) Entgegenhaltungen:
- WO-A1-2014/035471
- US-A1- 2011 015 610
- US-A1- 2013 331 774

## Beschreibung

Die vorliegende Erfindung betrifft eine Blutbehandlungskassette gemäß dem Oberbegriff des Anspruchs 1 und eine Blutbehandlungsvorrichtung gemäß dem Oberbegriff des Anspruchs 7.

Einmalteilsysteme werden in der Medizin- oder Labortechnik zunehmend als kompakte medizinische Funktionsvorrichtungen wie Kassettensysteme oder Blutbehandlungskassetten ausgeführt, in denen Flüssigkeiten und Gase, insbesondere medizinische Flüssigkeiten und Blut, in Kanälen und Kammern geführt werden. Sind sie zum einmaligen Gebrauch vorgesehen, so spricht man von Kassettendisposables oder Einwegkassetten.

Zumeist handelt es sich hierbei um Hartteil-Folie-Kassetten. Das Hartteil besteht regelmäßig aus einem Spritzgußwerkstoff wie beispielweise PE, PP, PA, ABS, PMMA, PC oder PVC. In ihm sind beispielsweise Schlauchanschlüsse, Konnektoren, Kammern, Kanäle und Zentriereinrichtungen ausgestaltet. Die Kammern und Kanäle sind in der Regel als fluidführende halboffene Strukturen ausgestaltet. Eine Folie aus zum Hartteil kompatiblen Werkstoffen (mit dem Hartteil verschweißbar oder verklebbar) verschließt die halboffenen Strukturen und komplettiert sie zu vollwertigen Kammern und Kanälen. Die Folie kann beispielweise nur an einem äußeren umlaufenden Rand mit der Blutbehandlungskassette verschweißt oder verklebt sein. Ebenfalls gibt es Ausführungen, bei denen die Berandungen der Kammern und Kanäle, die sog. Kanalrandstege, streifenförmig oder flächig mit der Folie verschweißt oder verklebt sind. Auf diese Weise entstehen Blutbehandlungskassetten, die vor dem Einbau in eine Behandlungsmaschine und nach dem Ausbau bereits eine definierte Fluidführung vorgeben.

Bestimmte Bereiche des Hartteils und der Folie werden häufig bewusst nicht miteinander verschweißt oder verklebt. Diese Bereiche können als Folienventile zwischen verschiedenen fluidführenden Bereichen genutzt werden. Zu diesem Zweck wird die Blutbehandlungskassette beim Einsetzen in die Blutbehandlungsvorrichtung zwischen eine Tür und eine Aktor-Sensor-Einheit der Blutbehandlungsvorrichtung eingebaut und anschließend die Tür durch ihr Schließen in eine sog. Verpress-Stellung gebracht, bei der die Folie gegen das Hartteil gepresst und die Blutbehandlungskassette mit der Folie räumlich definiert an die Aktor-Sensor-Matte der Aktor-Sensor-Einheit angekoppelt werden. In die Aktor-Sensor-Matte und Aktor-Sensor-Platte (oder -einheit) integrierte Aktoren (hierin auch als Aktuatoren bezeichnet) können Bewegungen durch die Folie hindurch ausüben, wodurch beispielsweise Pump- oder Ventil-Funktionen realisierbar sind. Mittels wenigstens eines, optional in der Aktor-Sensor-Platte vorgesehenen Sensors können beispielsweise Eigenschaften von Fluiden, welche die Blutbehandlungskassette durchströmen, gemessen werden.

Insbesondere bei Blutbehandlungskassetten mit kanalrandbündig aufgeschweißten Folien und Folienventilen kann es allerdings zu herstelltechnischen oder verfahrenstechnischen Problemen kommen.

Die Patentanmeldung US 2011/015610 A1 offenbart eine medizinische Fluidkassette mit einer Basis und einer Membran, die zusammen eine Fluidpumpkammer definieren.

WO2014035471 A1 offenbart eine weitere Blutbehandlungskassette gemäß dem Stand der Technik.

Eine erfindungsgemäße Aufgabe kann darin bestehen, eine weitere Blutbehandlungskassette vorzuschlagen. Ferner soll eine Blutbehandlungsvorrichtung zum Verwenden der Blutbehandlungskassette angegeben werden.

Die Aufgabe kann gelöst werden durch eine Blutbehandlungskassette mit den Merkmalen des Anspruchs 1, ferner durch eine Blutbehandlungsvorrichtung mit den Merkmalen des Anspruchs 7.

Erfindungsgemäß wird somit eine Blutbehandlungskassette mit einem als Hartteil ausgestalteten Kassettenkörper und einer Folie vorgeschlagen. Dabei ist die Folie mit dem Hartteil, beispielsweise durch Schweißen oder Kleben, verbunden und das Hartteil ist zumindest abschnittsweise gegenüber einem Äußeren durch die Folie be- oder abgedeckt, so dass aus Hartteil und Folie gemeinsam gebildete Kanäle oder Kammern oder Teile hiervon ausgestaltet werden. Ferner weist das Hartteil wenigstens einen Ventilsitz oder -abschnitt eines Ventils auf, hierin auch als Ventilgrund des Ventils bezeichnet.

Das Ventil ist ausgestaltet, um eine erste und eine hiervon verschiedene, zweite Stellung oder Ventilstellung einzunehmen. Dabei ist die erste Stellung eine geöffnete Stellung, in welcher das Ventil, insbesondere zum Gassterilisieren von Abschnitten des Hartteils, geöffnet ist. In der ersten Stellung berühren sich der Ventilgrund und ein Abschnitt der Folie, welcher im Gebrauch der Behandlungskassette über dem Ventilgrund angeordnet ist oder zu liegen kommt, nicht. Das Ventil ist konfiguriert und vorgesehen, um bei Aufbringen einer - insbesondere in Richtung auf den Ventilgrund hin und mittels einer auf den über dem Ventilgrund angeordneten Abschnitt der Folie wirkenden - Kraft in die zweite, geschlossene Stellung überzugehen. In der zweiten Stellung berühren sich der Ventilgrund und der Abschnitt der Folie, beispielsweise direkt oder indirekt. In der zweiten Stellung ist das Ventil geschlossen.

Des Weiteren weist die Blutbehandlungskassette, und insbesondere das Hartteil, wenigstens einen, insbesondere unter der Folie angeordneten, Abstandhalter auf. Der Abstandhalter ist federnd und/oder elastisch am Hartteil abgestützt und/oder mit diesem integral hergestellt. Dabei ist der Abstandhalter angeordnet, um in der ersten Stellung - und optional auch in der zweiten Stellung - Spannung, Kraft oder Druck auf den über dem Ventilgrund angeordneten Abschnitt der Folie auszuüben. Dabei ist der Abstandhalter integrales Element eines mit dem Hartteil verbundenen Federelements oder ist hieran angeordnet. Zudem sind auf dem Federelement Höcker angeordnet, welche von Drainagestrukturen durchzogen sind, und/oder das Federelement weist ein Funktionselement, nämlich ein integriertes Hydrophobmembran-Abstützgitter, eine Koagelfängerscheibe und/oder ein Rückschlagventil, auf.

Ferner wird eine erfindungsgemäße Blutbehandlungsvorrichtung vorgeschlagen, welche mit einer Blutbehandlungskassette verbunden ist und eine Aktor-Sensor-Platte aufweist. Die Aktor-Sensor-Platte weist wenigstens einen Aktuator auf, welcher wenigstens zwei, vorzugsweise mehr als drei, Teil-Aktuatoren aufweist. Diese sind derart angeordnet, um - vorzugsweise vollständig oder im Wesentlichen - unabhängig voneinander Kraft auf den Abschnitt der Folie des Ventils auszuüben.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale in beliebiger Kombination aufweisen.

In bestimmten erfindungsgemäßen, beispielhaften Ausführungsformen weist das Ventil nicht sowohl (oder nicht zugleich) einen Durchlass für eine Hauptströmung als auch einen hiervon unterscheidbaren Durchlass für eine Nebenströmung auf.

In einigen erfindungsgemäßen, beispielhaften Ausführungsformen berühren sich der Ventilgrund und ein über diesem angeordneter Abschnitt der Folie bei geöffneter Stellung des Ventils weder direkt noch indirekt.

In gewissen erfindungsgemäßen, beispielhaften Ausführungsformen ist das Ventil ausgestaltet, um mittels Druckaufbringung eines Aktors oder Aktuators einer Blutbehandlungsvorrichtung auf das Ventil, zu deren Betrieb die Blutbehandlungskassette bestimmungsgemäß mit der Blutbehandlungsvorrichtung verbunden wird, von der ersten Stellung in die zweite Stellung überführbar zu sein oder übergeführt zu werden.

In bestimmten erfindungsgemäßen, beispielhaften Ausführungsformen ist das Ventil als Folien- oder Phantomventil ausgestaltet.

In der erfindungsgemäßen Blutbehandlungskassette ist der Abstandhalter integrales Element eines mit dem Hartteil verbundenen Federelements oder ist hieran angeordnet.

In gewissen erfindungsgemäßen, beispielhaften Ausführungsformen ist das Federelement als Federbügel ausgestaltet oder weist einen solchen auf.

In einigen erfindungsgemäßen, beispielhaften Ausführungsformen der Blutbehandlungskassette ist der Abstandhalter das Federelement oder die nachstehend beschriebenen Höcker.

In bestimmten erfindungsgemäßen, beispielhaften Ausführungsformen weist das Federelement wenigstens eine Fixieröffnung oder -bohrung auf. Letztere ist vorgesehen, um das Federelement mittels wenigstens eines, in die wenigstens eine Bohrung einsteckbaren Fixierpins am Hartteil - vorzugsweise lösbar oder nicht lösbar - zu befestigen. In diesen oder in anderen erfindungsgemäßen, beispielhaften Ausführungsformen weist das Federelement wenigstens einen Fixierpin auf, vorgesehen, um das Federelement in wenigstens einer Fixieröffnung oder -bohrung des Hartteils - vorzugsweise lösbar oder nicht lösbar - zu befestigen.

In einigen erfindungsgemäßen, beispielhaften Ausführungsformen weist das Federelement wenigstens zwei Abstandhalter auf, welche wenigstens eine Drainagenut oder -vertiefung zwischen den Abstandhaltern aufweisen.

In gewissen erfindungsgemäßen, beispielhaften Ausführungsformen weist das Federelement, vorzugsweise in einem mittleren Bereich hiervon, ein Funktionselement, vorzugsweise ein integriertes Hydrophobmembran-Abstützgitter, eine Koagel- oder Gerinnselfängerscheibe und/oder ein Rückschlagventil auf.

In bestimmten erfindungsgemäßen, beispielhaften Ausführungsformen ist zwischen dem Federelement und dem Funktionselement wenigstens ein Spalt oder Spaltabschnitt oder eine Nut vorgesehen. Dies erlaubt eine zumindest geringfügige Relativbewegung zwischen einem Rahmenabschnitt oder Federbügel des Federelements einerseits und dem Abstützgitter andererseits. Einer gegenseitigen Beeinträchtigung hierbei ist hiermit vorteilhaft vorgebeugt.

In einigen erfindungsgemäßen, beispielhaften Ausführungsformen entspricht eine Absenkung des Ventilgrunds in Form einer Aussparung, Delle oder dergleichen gemessen am benachbarten Niveau des Kammer- oder Kanalrandstegs, hier auch als Dellentiefe des Ventilgrunds bezeichnet, dem 1- bis 3-fachen der Dicke oder Stärke der Folie. Alternativ kann der Ventilgrund hinter benachbarten Kanalrandstegen um das 1- bis 3-fache der Dicke oder Stärke der Folie in Richtung Inneres der Blutbehandlungskassette zurückgesetzt sein.
In gewissen erfindungsgemäßen, beispielhaften Ausführungsformen weist der Abstandhalter - bezogen auf die Richtung auf den Ventilgrund zu - eine gewölbte Oberkante auf. Ihre Wölbung beträgt in manchen erfindungsgemäßen, beispielhaften Ausführungsformen in vorgenannter Richtung das 1- bis 4-fache der Dicke oder Stärke der Folie.

In bestimmten erfindungsgemäßen, beispielhaften Ausführungsformen weist der Ventilgrund keine Hinterschnitte auf.

Die hierin beschriebenen Folienventile werden im Zusammenhang mit der vorliegenden Erfindung auch Phantomventile genannt, da sie im geschlossenen Zustand aus der Sicht der betroffenen Kanäle keine Veränderung des Strömungsraumes im Vergleich zu Kanalstellen oder Kammern ohne Folienventil darstellen. Sie werden mit Blick auf den Strömungsraum - gleich einem Phantom - nicht wahrgenommen.

In bestimmten beispielhaften erfindungsgemäßen Ausführungsformen weist der Abstandhalter, hierin auch als Beabstandungsvorrichtung bezeichnet, Höcker auf, welche den über ihnen liegenden Abschnitt der Folie lokal durch direkten oder indirekten Kontakt mit dieser ausbeulen. Auf diese Weise schaffen sie einen Abstand des betreffenden Folienabschnitts zu einem benachbarten Kanalsteg und/oder dem Ventilgrund. Die Höcker ragen dabei in manchen erfindungsgemäßen Ausführungsformen über die allgemeine Ebene der flachen Folie hinaus, also über jene Ebene, in welcher die Folie im Wesentlichen plan auf dem Hartteil aufliegt, beispielsweise über die Verklebe- oder Verschweißebene. Dies kann vorteilhaft einen Sicherheitsabstand schaffen, welcher ein zuverlässiges Durchströmen des offen gehaltenen Ventils erlaubt.

In einigen beispielhaften erfindungsgemäßen Ausführungsformen sind die Höcker des Abstandhalters angeordnet, um die Folie lokal in der allgemeinen Ebene der flachen Folie zu halten. Da die Höcker oder der Abstandhalter dabei die Folie im Bereich des Ventils nicht über die allgemeine Ebene hinaus anheben, kann hierbei vorteilhaft eine ungewollte und möglicherweise irreversible Dehnung des Folienabschnitts verhindert werden.

In bestimmten beispielhaften erfindungsgemäßen Ausführungsformen sind die Höcker federnd und/oder nachgiebig auf dem Abstandhalter angeordnet.

In einigen beispielhaften erfindungsgemäßen Ausführungsformen weist der Abstandhalter neben den Höckern ein weiteres, in der Blutbehandlungskassette enthaltenes Funktionselement auf.

In bestimmten beispielhaften erfindungsgemäßen Ausführungsformen sind die Höcker (auch als Erhebungen zu bezeichnen) des Abstandhalters in wenigstens einer Reihe parallel zum Verlauf des Dichtsitz-Steges oder Ventilgrunds angeordnet. Diese Anordnung kann die Folie vorteilhaft auch dann auf aus strömungstechnischer Sicht ausreichendem Abstand vom Dichtsitz-Steg oder vom Ventilgrund halten, wenn die Höcker ausgestaltet sind, um nur geringfügig über die Kanalrandstege, mit welchen die Folie verbunden ist, überzustehen. Der Strömungsquerschnitt wird über die Länge des mittels des Abstandhalters erzeugten Durchlasses zwischen Ventilgrund und Folie erzeugt.

In bestimmten beispielhaften erfindungsgemäßen Ausführungsformen sind die Höcker von Drainagestrukturen zwischen und/oder auf den Höckern durchzogen oder versehen. Letztere verringern eine Kontaktfläche zwischen Abstandhalter und Folie während der Sterilisation und können hierdurch vorteilhaft zu einer erhöhten Wirksamkeit des Sterilisationsprozesses beitragen.

In manchen beispielhaften erfindungsgemäßen Ausführungsformen sind die Höcker auf einem oder mehreren Federelementen angeordnet.

In bestimmten beispielhaften erfindungsgemäßen Ausführungsformen weist der Abstandhalter Höcker auf, welche den Federweg in Richtung auf das Hartteil begrenzen. Auf diese Weise kann eine Überlastung des Federelements vorteilhaft vermieden werden. Diese Höcker oder Erhebungen können beispielsweise an einer Unterseite oder seitlichen Fläche des Federelements angeordnet sein. Sie können als Anschläge oder ihrerseits als Abstandhalter (für das Federelement, nicht für die Folie) dienen.

In manchen beispielhaften erfindungsgemäßen Ausführungsformen ist der Abstandhalter angeordnet, um einen Abstand des Folienabschnitts vom Ventilgrund sicherzustellen, welcher einen Gas- oder Dampfaustausch über das Ventil hinweg, also zwischen Folie und Ventilgrund, zulässt.

In bestimmten beispielhaften erfindungsgemäßen Ausführungsformen wirkt der Abstandhalter von einem Inneren der Blutbehandlungskassette, also einem Raum zwischen Hartteil und Folie, auf die Folie ein.

In manchen beispielhaften, erfindungsgemäßen Ausführungsformen weist die Blutbehandlungsvorrichtung einen unterteilten oder lamellierten Anpressstempel als Aktor auf. Die Unterteilung oder Lamellierung erlaubt es dem Aktor auf Krümmungen des Ventilgrunds einzugehen.

In bestimmten beispielhaften erfindungsgemäßen Ausführungsformen weist der unterteilte oder lamellierte Anpressstempel eine reibungsfreie Festkörpergelenk-Lagerung und/oder eine Federunterstützung auf.

In einigen beispielhaften erfindungsgemäßen Ausführungsformen weist die Blutbehandlungsvorrichtung anstelle des oder ergänzend zum lamellierten Anpressstempel mit vorzugsweise reibungsfreier Festkörpergelenk-Lagerung und Federunterstützung andere Toleranzausgleichsvorrichtungen in der Aktor-Sensor-Einheit auf. Zu ihnen zählen strukturierte oder inseitig gehöckerte Aktor-Sensor-Matten, strukturierte oder gehöckerte Aktor-Sensor-Platten, zwischengeschaltete Schaumstoffe und Druck-Kissen, strukturierte zwischengeschaltete Elastomerkörper und dergleichen.

In manchen beispielhaften, erfindungsgemäßen Ausführungsformen sind die hierin beschriebenen Folienventile solche, welche auch während des Gebrauchs der Blutbehandlungskassette eine Funktion einnehmen. In anderen beispielhaften erfindungsgemäßen Ausführungsformen dienen die Folienventile allein als Ventile, mit denen vor Gebrauch der Blutbehandlungskassette ein Übertritt von Sterilisationsmittel zwischen Kammer und Kanal/Kammer sichergestellt werden soll. In diesen Ausführungsformen bleibt das Folienventil nach Beginn der Blutbehandlungssitzung mittels Aktuator verschlossen.

Einige oder alle erfindungsgemäßen Ausführungsformen können einen oder mehrere der oben oder im Folgenden genannten Vorteile aufweisen.

Beim Sterilisieren der Blutbehandlungskassetten mit Gasen oder mit Dampf finden zum Gasaustausch in der Regel mehrere Wechsel zwischen Vakuum- und Überdruck-Phasen statt. Dabei wirken die verbleibenden, nicht verschweißten oder verklebten Dichtsitzbereiche der Ventile als Strömungswiderstände. Bei der Umschaltung von Vakuum auf Überdruck wirken sie gelegentlich und unbeabsichtigt sogar als Rückschlagventil, wenn sich die Folie wegen des in der Blutbehandlungskassette erst verzögert hergestellten Druckausgleichs in Richtung auf die Kavitäten und Kammern der Blutbehandlungskassette verformt. Dabei schließen sich die Folienventile möglicherweise und können dadurch den Zutritt von Sterilisationsgasen in einzelne Kanäle oder Kammern be- oder gar verhindern, weshalb es zu unzureichender Sterilisierung kommen kann. Zum Lösen dieses Problems musste bislang auf unwirtschaftliche Sterilisationsverfahren zurückgegriffen werden. Diese Nachteile können mittels der erfindungsgemäßen Blutkassette verringert oder vermieden werden, denn ihre Folienventile bleiben Dank des Abstandhalters beim Sterilisieren selbst bei Anlegen von Unterdruck offen.

Aufgrund des erfindungsgemäß vorgesehenen Abstandhalters, welcher die Folie über den Folienventilen, an welchen diese nicht mit dem Hartteil verschweißt sind, unter Spannung hält, können die aufgrund der Wechsel von Vakuum zu Überdruck und umgekehrt auftretenden mechanischen Beanspruchungen der nicht verschweißten Folieabschnitte verhindert oder zumindest vermindert werden. Dies kann auch einem sich aufgrund der mechanischen Beanspruchungen ergebenden Durchhängen der Folien entgegenwirken.

Erfindungsgemäß wird somit eine technische Lösung vorgeschlagen, die es vorteilhaft erlaubt, die Folie an einer Stelle oder an mehreren Stellen der Berandungen der Kammern und Kanäle vom Hartteil zu beabstanden, so dass analog zur Funktionsweise der bereits beschriebenen Phantomventile eine fluidische Verbindung zwischen Kammern und Kanälen zustande kommt und damit der Transport der Sterilisationsgase verbessert und die bei den Druckwechseln entstehenden Folienbelastungen reduziert werden.

Zu den weiteren Vorteilen zählt der erfindungsgemäß mögliche Toleranzausgleich, welcher durch die federnde Lagerung des Abstandhalters aber auch durch die mehrteilige oder lamellierte Ausgestaltung des Aktuator zum Schließen des Folienventils möglich ist. Der Aktuator der erfindungsgemäßen Blutbehandlungsvorrichtung dient dabei als Toleranzausgleichsvorrichtung für gedellte, also nicht gerade verlaufende Folien-Dichtsitz-Stege, bei denen ein Einbautoleranz- und Formtoleranz-Ausgleich in Richtung längs des Dichtsitz-Steges beispielsweise über unabhängig bewegliche Lamellen gewährleistet wird.

Die Toleranzausgleichsvorrichtungen mittels der Aktor-Sensor-Einheit können dabei vorteilhaft die Dichtwirkung von flachen oder gedellten Folienventilen aufrecht erhalten und sind robust gegenüber Maßtoleranzen der Blutbehandlungskassette, der Blutbehandlungsvorrichtung, oder die Ausrichtung zwischen Blutbehandlungskassette und Blutbehandlungsvorrichtung betreffend.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil vereinfachten Figuren gilt:
- Fig. 1: zeigt eine Seitenansicht einer an ihrer Vorderseite mit einer Abdeckungseinrichtung versehenen erfindungsgemäßen Blutbehandlungskassette gemäß einer bevorzugten Ausführungsform;
- Fig. 2: zeigt die externe Funktionseinrichtung der Fig. 1 mit nach zerstörendem Aufschneiden aufgeklappter Abdeckungseinrichtung;
- Fig. 3: zeigt die Blutbehandlungskassette aus Fig. 1 und Fig. 2 von ihrer Rückseite;
- Fig. 4: zeigt perspektivisch einen Ausschnitt eines Hartteils einer Blutbehandlungskassette;
- Fig. 5a,b: zeigen perspektivisch ein Federelement der Blutbehandlungskassette;
- Fig. 6: zeigt eine Draufsicht auf den in der Fig. 4 dargestellten Ausschnitt einer Blutbehandlungskassette mit eingebautem Federelement;
- Fig. 7: zeigt eine Delle eines Ventils der vorangegangenen Figuren;
- Fig. 8: zeigt einen Schnitt durch die noch nicht aufgerüstete Blutbehandlungskassette der Fig. 6 entlang der Linie B-B;
- Fig. 9: zeigt einen Schnitt durch die noch nicht aufgerüstete Blutbehandlungskassette der Fig. 6 entlang der Linie D-D;
- Fig. 10: zeigt eine Ausführungsform einer in eine erfindungsgemäße Blutbehandlungsvorrichtung eingelegten und von dieser verpressten Blutbehandlungskassette in Draufsicht;
- Fig. 11: zeigt eine Ausführungsform einer in eine erfindungsgemäße Blutbehandlungsvorrichtung eingelegten und von dieser verpressten Blutbehandlungskassette in Schnittansicht entlang der Linie D-D der Fig. 10; und
- Fig. 12: zeigt eine Ausführungsform einer in eine erfindungsgemäße Blutbehandlungsvorrichtung eingelegten und von dieser verpressten Blutbehandlungskassette in Schnittansicht entlang der Linie B-B der Fig. 10.

Die normalen Pfeile in den Figuren zeigen die Richtung des Blutstroms an. Die Blockpfeile zeigen jeweils die Richtung des Substituatstroms an.

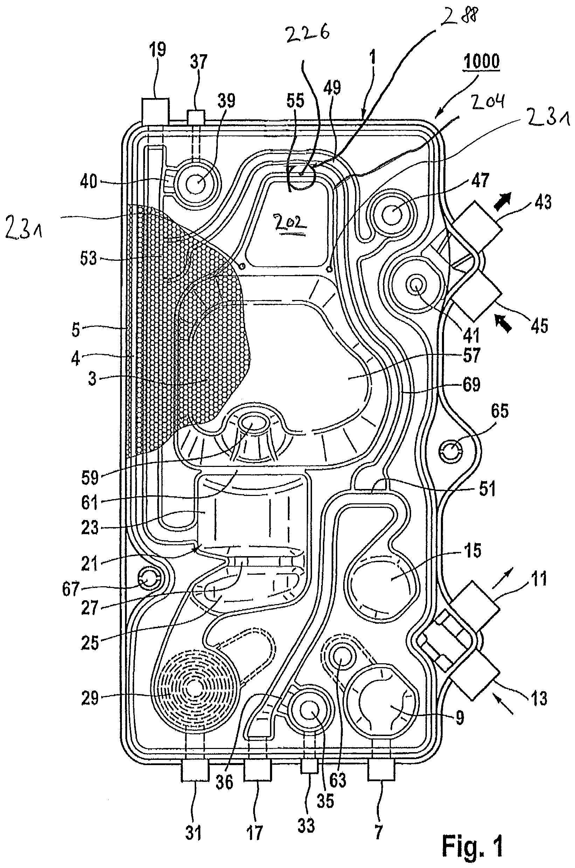

**Fig. 1** zeigt eine Seitenansicht einer erfindungsgemäßen Blutbehandlungskassette 1000, welche an der Oberfläche, auf die in Fig. 1 geblickt wird, mit einer Abdeckungseinrichtung versehen ist.

Die erfindungsgemäße Blutbehandlungskassette 1000 wird im Folgenden auch kurz als Kassette 1000 bezeichnet.

Die Kassette 1000 weist ein Hartteil 1 auf. Wie in Fig. 1 exemplarisch gezeigt, weist das Hartteil 1 Kammern, Kanäle und Ventile auf. Wie in Fig. 1 exemplarisch weiter gezeigt ist, sind die Kammern, Kanäle und Ventile in das Hartteil 1 integriert bzw. zumindest teilweise vom Hartteil 1 ausgestaltet.

Die Kassette 1000 der Fig. 1 ist an ihrer Vorderseite mit einer Abdeckungseinrichtung, hier beispielsweise einer Folie 3, versehen. Die Abdeckungseinrichtung kann eben, d. h. plan, auf das Hartteil 1 aufgeschweißt sein.

Eine Ausgestaltung mit einer dreidimensionalen Ausführung der Schweiß- und Dichtkontur ist erfindungsgemäß ebenfalls möglich.

Die Abdeckungseinrichtung kann die Kammern und/oder Kanäle des Hartteils 1 der Kassette 1000 verschließen, und zwar gegenüber einer dem Hartteil 1 abgewandten Seite der Abdeckungseinrichtung und/oder gegenüber der Atmosphäre.

Wie in Fig. 1 zu erkennen ist, liegt die Folie an einem umlaufenden Dichtsteg 4 auf dem Hartteil 1 der Kassette 1000 auf. Die Folie 3 ist an einer umlaufenden Schweißnaht 5 mit dem Hartteil 1 der Kassette 1000 verschweißt.
Der umlaufende Dichtsteg kann alternativ freiliegend ausgeführt werden.

Die Folie 3 kann an weiteren lokalen Verschweißungen (nicht gezeigt) mit dem Hartteil 1 der Kassette 1000 verbunden sein. Diese können ebenfalls umlaufend, also geschlossen im Sinne einer abschließenden Begrenzung ähnlich einem Ring, und/oder punktförmig sein.

Die Folie 3 kann an lokalen Stellen punkt- oder linienförmig mit dem Hartteil der Kassette 1000 verbunden, z. B. verschweißt, sein, insbesondere an den Randzonen der flüssigkeitsführenden Kanäle.

Die Folie 3 kann durch Laser-Schweißen mit dem Hartteil der Kassette 1000 verbunden werden. Dabei ist es vorteilhaft, wenn der lokale Hitzeeintrag unter Verwendung einer Licht absorbierenden Komponente erfolgt. Die Licht absorbierende Komponente kann Bestandteil des Materials der Folie und/oder des Hartteils sein oder eine Schicht, die zwischen Folie und Hartteil oder über der Folie angeordnet ist. Die Schicht kann eine Folienschicht sein.

Die Kassette 1000 kann wenigstens mit der in Fig. 1 gezeigten Vorderseite an eine Blutbehandlungsvorrichtung (in Fig. 1 nicht gezeigt) angekoppelt werden. Eine beispielhafte Vorgehensweise zum geeigneten Ankoppeln einer Kassette 1000 an eine Ankoppelfläche einer Blutbehandlungsvorrichtung ist in den Patentanmeldungen 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, und 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die ebenfalls am 10. März 2009 beim DPMA eingereicht wurde, beschrieben. Die Kassette 1000 kann mit der Ebene der Folie 3 - oder über diese - an eine Ankoppelfläche der Blutbehandlungsvorrichtung angekoppelt werden. Die Ankoppelfläche kann vorzugsweise dreidimensional ausgeführt sein.

Die Ankoppelfläche der Blutbehandlungsvorrichtung kann zum Beispiel an einem in Fig. 1 oberen Abschnitt hiervon um 8° gegen eine in Fig. 1 von oben nach unten verlaufende Senkrechte nach hinten (in der Fig. 1 die sich vom Betrachter in die Zeichenebene hinein erstreckende Richtung) geneigt sein.

Die Kassette 1000 weist einen arteriellen Patientenanschluss 7 auf.

Die Kassette 1000 weist eine arterielle Druckmesskammer 9 auf. Diese kann entsprechende Sensoren aufweisen. Die Sensoren können bevorzugt über eine Verkabelung Signale übermitteln. Die Sensoren können aber auch so ausgeführt sein, dass sie kabellos Signale übermitteln.

Die Kassette 1000 weist einen Konnektor 11 für den Blutaustritt aus der Kassette 1000 sowie einen Konnektor 13 für den Bluteintritt in die Kassette 1000 auf.
Die beiden Konnektoren 11 und 13 sind mit einem Pumpschlauchsegment oder -set einer Blutpumpe verbindbar.

Die Kassette 1000 weist ferner eine Kammer 15 mit einer Druckmessstelle zur Druckmessung im extrakorporalen Blutkreislauf vor dem Dialysator ("Prä-Filter") bzw. nach der Pumpe ("Post-Pumpe") auf.

An der Kammer 15 kann durch die Folie 3 hindurch bzw. über die Folie 3 der Druck im extrakorporalen Kreislauf vor dem Dialysator gemessen werden.

Die Kassette 1000 weist eine arterielle Filterleitung 17 sowie eine venöse Filterleitung 19 auf.

Das Innere der Kassette 1000 weist eine venöse Blutkammer 21 auf. Die venöse Blutkammer 21 ist in einen oberen Raum 23 und einen unteren Raum 25 unterteilt.

Der obere Raum 23 der venösen Blutkammer 21 kann eine seitlich tangentiale Bluteinströmung zulassen. Dabei kann Blut seitlich durch den Einlass (der linken Seite in Fig. 1) in den oberen Raum 23 einströmen und sich tangential zu den Wänden des oberen Raums 23 ausbreiten. Eine seitlich tangentiale Bluteinströmung kann eine Zone mit im Wesentlichen oder vollständig stabiler Rotationsströmung des Bluts in dem oberen Raum 23 der venösen Blutkammer 21 erzeugen.

Der untere Raum 25 der venösen Blutkammer 21 kann eine Beruhigungszone für die Blutströmung darstellen. Es ist möglich, dass in einer derartigen Beruhigungszone im Wesentlichen keine oder überhaupt keine Rotationsströmung des Bluts vorliegt.

Die venöse Blutkammer 21 ist durch eine Querschnittsverjüngung 27 des Hartteils 1 der Kassette 1000 in den oberen Raum 23 und den unteren Raum 25 aufgeteilt. Die Querschnittsverjüngung 27 reduziert den Querschnitt der venösen Blutkammer 21 derart in seiner Breite und Tiefe, dass sich eine Strömungsschnelle ergibt, nach deren Durchquerung ein die venöse Blutkammer 21 der Kassette 1000 durchströmendes Fluid eine langsamere Strömungsgeschwindigkeit annimmt. Der obere Raum 23 und der untere Raum 25 stehen in Fluidkommunikation.

Durch eine derartige Konstruktion, d. h. einer Aufteilung der venösen Blutkammer 21 in eine Zone mit im Wesentlichen oder vollständig stabiler Rotationsströmung des Bluts und in eine Beruhigungszone für die Blutströmung, kann vorteilhaft eine effiziente Abscheidung für Luft aus dem Blut oder Fluid erreicht werden.

Wandungen des oberen Raums 23 und des unteren Raums 25 der venösen Blutkammer 21 können in geeigneter Weise einer Neigung des oberen Abschnitts der Kassette 1000 in Fig. 1 gegen die Vertikale, beispielsweise einer Neigung des oberen Teils der in Fig. 1 gezeigten Kassette 1000 um 8° nach hinten (in die Zeichenebene hinein), angepasst werden. Sie können in geeigneter Weise derart gerundet ausgestaltet sein, dass sie vorteilhaft eine strömungsoptimierte Berührfläche für Fluide darstellt, welche die venöse Blutkammer 21 durchströmen.

Die Kassette 1000 weist einen Gerinnselfänger 29 auf.
Als Gerinnselfänger kann vorzugsweise ein Gerinnselfänger verwendet werden, wie er in der von der Anmelderin der vorliegenden Erfindung in der Patentanmeldung 10 2009 024 495.6 mit dem Titel *"Gerinnselfänger, externe Funktionseinrichtung, Blutkreislauf sowie Behandlungsvorrichtung",* die am 10. Juni 2009 beim DPMA eingereicht wurde, offenbart wurde.

Am Gerinnselfänger 29 kann durch die Folie 3 hindurch bzw. über die Folie 3 der Druck im extrakorporalen Kreislauf gemessen werden, also insbesondere nach Durchlaufen des Dialysators.

Die Kassette 1000 weist einen venösen Patientenanschluss 31 auf.

Die Kassette 1000 weist eine arterielle Heparin-Zugabestelle 33 auf. Dabei gilt zu beachten, dass die Heparin-Zugabestelle 33 (wie auch eine venöse Heparin-Zugabestelle 37) auch zur Zugabe anderer pharmakologischer Wirkstoffe als Heparin, welche nur vorzugsweise Antikoagulantien sind, oder Wirkstoffkombinationen geeignet und vorgesehen sein kann. Dies ist stets auch dann zu beachten, wenn zuvor oder im Folgenden von Heparin in beliebigem Zusammenhang die Rede ist.

Die Kassette 1000 weist ein Rückschlagventil 35 der arteriellen Heparin-Zugabestelle 33 auf.

Beispielhafte Rückschlagventile zum Einsatz als Rückschlagventil 35 der arteriellen Heparin-Zugabestelle 33 sowie als weitere Rückschlagventile der Kassette 1000 sind in der Patentanmeldung 10 2009 024 469.7 der Anmelderin der vorliegenden Erfindung mit dem Titel *"Ventilvorrichtung, Ventileinsatz, externe Funktionseinrichtung, Behandlungsvorrichtung sowie Verfahren",* die am 10. Juni 2009 beim DPMA eingereicht wurde, offenbart.

Die Kassette 1000 weist ein arterielles Heparin-Zugabeventil 36 auf. Mit Hilfe des arteriellen Heparin-Zugabeventils 36 kann die Zugabe von Heparin in die arterielle Filterleitung 17 gesteuert oder geregelt werden.

Das arterielle Heparin-Zugabeventil 36 kann als so genanntes Phantomventil ausgestaltet sein.

Der Begriff "Phantomventil", wie er hierin verwendet wird, bezeichnet ein Element mit einer mittels Aktor erreichbaren Aktor-Fläche (hier beispielsweise eine Aktor-Membran), welches die Funktion eines Ventils übernehmen kann.

Die Aktor-Membran ist unter Aufbringung einer Kraft hierauf, z. B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.

Wenn die Kraft wieder von der Aktor-Membran genommen wird, kann diese in beispielsweise eine Grundposition, z. B. einen nicht gewölbten Zustand, zurückkehren.

Ein Phantomventil zur Verwendung als arterielles Heparin-Zugabeventil 36 sowie weitere Phantomventile der Kassette 1000 können mit oder aus einem Stegabschnitt eines Kanals am Hartteil 1 der Kassette 1000 und einem dem Stegabschnitt an- oder gegenüberliegenden Abschnitt der Folie 3 ausgestaltet werden.

Phantomventile können durch Aktoren der Blutbehandlungsvorrichtung betätigt werden.

Zum Schließen eines Phantomventils kann der Abschnitt der Folie 3 auf den Stegabschnitt gedrückt werden. Zum Öffnen des Phantomventils kann der Abschnitt der Folie 3 wieder vom Stegabschnitt abgehoben werden.

Weitere Beispiele und/oder Ausführungsformen für Phantomventile können der Patentanmeldung 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die am 10. März 2009 von der vorliegenden Anmelderin beim DPMA eingereicht wurde, sowie der DE 100 53 441 A1 und der DE 102 24 750 A1 entnommen werden.

Die Kassette 1000 weist eine venöse Heparin-Zugabestelle 37 auf. Die venöse Heparin-Zugabestelle 37 kann als Luer-Konnektor ausgestaltet sein.

Die Kassette 1000 weist ein Rückschlagventil 39 der venösen Heparin-Zugabestelle 37 auf.

Die Kassette 1000 weist ein venöses Heparin-Zugabeventil 40 auf. Mit Hilfe des venösen Heparin-Zugabeventils 40 kann die Zugabe von Heparin in die venöse Filterleitung 19 gesteuert oder geregelt werden.

Die Kassette 1000 weist eine Substituat-Zugabestelle 41 bzw. einen Substituatkonnektor auf.

Die Substituat-Zugabestelle 41 kann eine Verbindungseinrichtung sein, wie sie in der Patentanmeldung 10 2009 024 575.8 der vorliegenden Anmelderin beschrieben ist, welche mit dem Titel *"Verbindungseinrichtung und Verfahren zum Konnektieren wenigstens zweier fluidführender medizintechnischer Systeme, sowie medizintechnische Vorrichtung"* am 10. Juni 2009 von der vorliegenden Anmelderin beim DPMA eingereicht wurde.

Die Substituat-Zugabestelle 41 kann mit einem Berührschutzelement (nicht gezeigt) versehen sein. Die Substituat-Zugabestelle 41 kann mit einem Tropfschutz (nicht gezeigt) versehen sein. Der Tropfschutz kann durch eine integrierte Verschlusshülse realisiert werden. Der Tropfschutz kann ein Heraustropfen von Resten von Substituat und/oder Blut beim Lösen der Kassette 1000 und anschließendem Entnehmen der Kassette 1000 aus der Blutbehandlungsvorrichtung verhindern.

Der Tropfschutz kann abnehmbar ausgeführt sein. Er kann als Haube oder Deckel ausgestaltet sein.

Die Substituat-Zugabestelle 41 oder ein anderer Abschnitt der Kassette 1000 kann ferner einen Originalitätsschutz bieten, durch welchen der Anwender ohne Mühe oder auf einen Blick erkennt, ob die Kassette 1000 bereits benutzt wurde. Dieser Originalitätsschutz kann mittels des Berührschutzelements, der Verschlusshülse oder einer anderen Struktur realisiert sein. Die entsprechende Struktur kann vorzugsweise ihre Position innerhalb der oder bezogen auf die Kassette 1000 erkennbar verändern. Sie kann vorzugsweise ihre Gestalt verändern.

Zudem kann die Substituat-Zugabestelle 41 oder ein anderer Abschnitt der Kassette 1000 einen Wiederverwendungsschutz bzw. Schutz gegen ein Wiederverwenden bieten. Vorzugsweise durch eine Verschlusshülse wird die Kassette 1000 - vorzugsweise irreversibel - im Hinblick auf den Versuch einer Wiederverwendung unbrauchbar gemacht. Soll die Kassette 1000 trotzdem erneut verwendet werden wollen, so messen Sensoren der Blutbehandlungsvorrichtung nicht die Signalverläufe, die bei Verwenden einer neuen Kassette gemessen werden würden.

Dies kann hierauf beruhen, dass keine Flüssigkeit in die Kassette 1000 oder in die Substituat-Zugabestelle 41 gelangen kann oder wenigstens nicht in ausreichender oder üblicher Menge. Die Steuereinheit der Blutbehandlungsvorrichtung kann dies erkennen. Es kann eine Warnung veranlasst werden.

Als Originalitätsschutz oder Wiederverwendungsschutz kann vorzugsweise ein Originalitätsschutz oder ein Wiederverwendungsschutz verwendet werden, wie er in der von der Anmelderin der vorliegenden Erfindung in der Patentanmeldung 10 2009 024 575.8 mit dem Titel *"Verbindungseinrichtung und Verfahren zum Konnektieren wenigstens zweier fluidführender medizintechnischer Systeme, sowie medizintechnische Vorrichtung",* die am 10. Juni 2009 beim DPMA eingereicht wurde, offenbart wurde.

Die Kassette weist einen Konnektor 43 für einen Substituataustritt aus der Kassette 1000 sowie einen Konnektor 45 für einen Sustituateintritt in die Kassette 1000 auf.

Die Konnektoren 43 und 45 sind mit einem Pumpschlauchsegment oder -set einer Substituatpumpe verbindbar.

Die Kassette 1000 weist ein Rückschlagventil 47 für Substituatzugabe auf.

Über Betätigung des Rückschlagventils 47 kann Substituat in eine Substituatleitung 49 eingebracht werden.

Die Kassette 1000 weist ein Prädilutions-Zugabeventil 51 auf. Das Prädilutions-Zugabeventil 51 kann als Phantomventil ausgestaltet sein.

Die Kassette 1000 weist ein Postdilutions-Zugabeventil 53 auf. Das Postdilutions-Zugabeventil 53 kann als Phantomventil ausgestaltet sein.

Die Kassette 1000 weist eine Single-Needle-Sterilmembran 55 auf.

Die Kassette 1000 weist eine Single-Needle-Kammer 57 auf. Die Single-Needle-Kammer 57 ist in Fig. 1 oberhalb der venösen Blutkammer 21 angeordnet.
Im Inneren der Single-Needle-Kammer 57 ist ein Blutschwallumleitungselement 59 angeordnet. Das Blutschwallumleitungselement 59 kann dem Abbremsen eines Blutschwalls und/oder dem Auslöschen dessen Impulses dienen.

Eine Verbindung mit einem Inneren der Single-Needle-Kammer 57 kann mittels einer Verbindungseinrichtung vorgesehen sein, wie sie in der von der Anmelderin der vorliegenden Erfindung mit dem Titel *"Einrichtung sowie externe Funktionseinrichtung und Behandlungsvorrichtung zum Behandeln von medizinischen Fluiden"* unter 10 2009 024 467.0 am 10. Juni 2009 beim DPMA eingereichten Patentanmeldung offenbart wurde.

Die Kassette 1000 weist ein Single-Needle-Blutventil 61 auf. Das Single-Needle-Blutventil 61 kann als Phantomventil ausgestaltet sein.

Die Kassette 1000 weist eine Absaugstelle 63 auf. Die Absaugstelle 63 kann zur Vakuumankopplung der Kassette 1000 an die Blutbehandlungsvorrichtung dienen, wie dies beispielsweise in der Patentanmeldung DE 10 2007 042 964 A1 mit dem Titel *"Vorrichtung und Verfahren zur Behandlung einer medizinischen Flüssigkeit",* die am 10. September 2007 beim DPMA eingereicht wurde, beschrieben ist.

Die Kassette 1000 weist ein primäres Ausrichtungszentrum 65 auf. Das primäre Ausrichtungszentrum 65 kann vorteilhaft zum Ausrichten und/oder Verrasten der Kassette 1000 an der Blutbehandlungsvorrichtung dienen.

Die Kassette 1000 weist eine sekundäre Ausrichtungsstelle 67 auf. Die sekundäre Ausrichtungsstelle 67 kann zum Ausrichten und/oder Verrasten der Kassette 1000 an der Blutbehandlungsvorrichtung dienen.

Die Kassette 1000 ist vor Beginn des Primens mit Gas (z. B. steriler Luft) gefüllt. Beim Primen des extrakorporalen Blutkreislaufs muss diese Gasfüllung verdrängt werden. Eine Blutbehandlungskassette stellt hierbei allgemein eine besondere Herausforderung dar, weil es sowohl aufsteigende als auch fallende Leitungen und zudem Kammern gibt, in denen keine "Luftnester" verbleiben dürfen. Die vorliegende Kassette 1000 weist zu diesem Zweck besondere Konstruktionsmerkmale auf:
Die Kammer 15 zum Messen des arteriellen Drucks ist derart konstruiert, dass die gesamte Luft in ein Pumpschlauchsegment (z. B. in das Pumpschlauchsegment) aufsteigen kann. Es gibt hierbei vorteilhaft keine Toträume. Luft, die aus der arteriellen Druckmesskammer in das Pumpschlauchsegment der Blutpumpe selbständig aufsteigt, wird ab dem Eingriffsbereich der Blutpumpe (z. B. durch die Rollen einer Rollenpumpe) durch das Pumpschlauchsegment zwangsgefördert. Sobald die Pumpe keinen Einfluss mehr hat (weil z. B. die Rollen in Ausgriff gehen) steigt die Luft selbsttätig in Förderrichtung in die Kassette 1000 auf.

Die venöse Rückführleitung (bzw. ein venöser Abschnitt des extrakorporalen Kreislaufs) ist eine Fallleitung. Ab einem gewissen darin herrschenden Volumenstrom (z. B. 200 ml/min bei der in Fig. 1 gezeigten Kassette 1000) werden Luftblasen im Blut auch entgegen der Erdbeschleunigung "mitgerissen". Dieser Effekt wird bei den Fallleitungen ausgenutzt. Die Leitungsquerschnitte der Fallleitungen sind so klein ausgelegt, dass durch die Strömungsgeschwindigkeit eine Zwangsförderung der Luftblasen auch entgegen der Erdbeschleunigung funktioniert.

In der venösen Blutkammer 21 sind große Querschnitte vorgesehen, derart, dass aufgrund der dort langsamen bzw. niedrigen Strömungsgeschwindigkeiten Luftblasen verlässlich entgegen der Hauptströmungsrichtung aufsteigen können.

Weitere konstruktive Besonderheiten der Kassette 1000 sind:
Die Phantomventile 40, 51 und 53 sind räumlich derart ausgerichtet, dass Blut (welches eine höhere Dichte als Wasser bzw. Substituat etc. hat) bei Betrieb der Kassette 1000 mit Blut kaum "nach oben" oder "zur Seite" in geöffnete Phantomventile eindringen kann, weil es gegenüber dem leichteren Wasser absinkt. Eine solch vorteilhafte Ausrichtung ist durch die Phantomventile 40, 51, und 53 realisiert. Beim Ventil 36 besteht hingegen kein solches Erfordernis, d.h. dort kommt es auf die Orientierung nicht an.

Der Leitungskanal (Stichkanal) unter dem Rückschlagventil 47 für Substituatzugabe ist aus demselben Grunde aufsteigend konstruiert. Im Falle einer Fehlfunktion der Prä- und/oder Postdilutionsventile 51 und 53 und einer daraus resultierenden Blut-Bypassströmung kann kein Blut in die Substituatleitung 49 aufsteigen. Vielmehr strömt das Blut an der Mündung der entsprechenden Stichleitung vorbei.

Die Neigung der Kassette 1000 beträgt vorzugsweise 5° bis 11°, besonders bevorzugt die bereits genannten 8°.
Das Bezugszeichen 288 bezeichnet ein Phantomventil, welches in einer ersten Stellung einen Einstrom in die Kammer 202 ermöglicht oder in einer zweiten Stellung unterbindet. In seiner einfachsten, von der vorliegenden Erfindung umfassten Ausgestaltung ist ein Phantomventil ein Folienventil, bei welchem ein Fluidweg zwischen Hartteil 1 und darüberliegendem Abschnitt der Folie 3 durch vorübergehendes Anpressen der Folie 3 auf einen Ventilgrund, etwa einen Steg oder anderen Abschnitt des Hartteils 1, unterbunden und nach Aufheben der anpressenden Kraft wieder geöffnet wird.

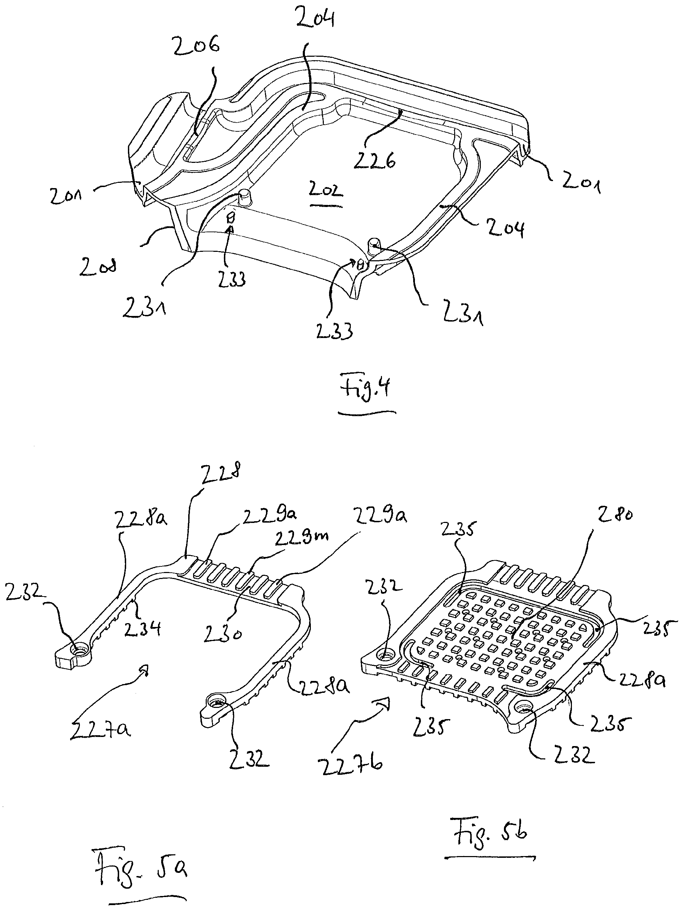

Die Bezugszeichen 202, 204, 226 und 231 werden mit der Beschreibung der Fig. 4 bis 12 erläutert.

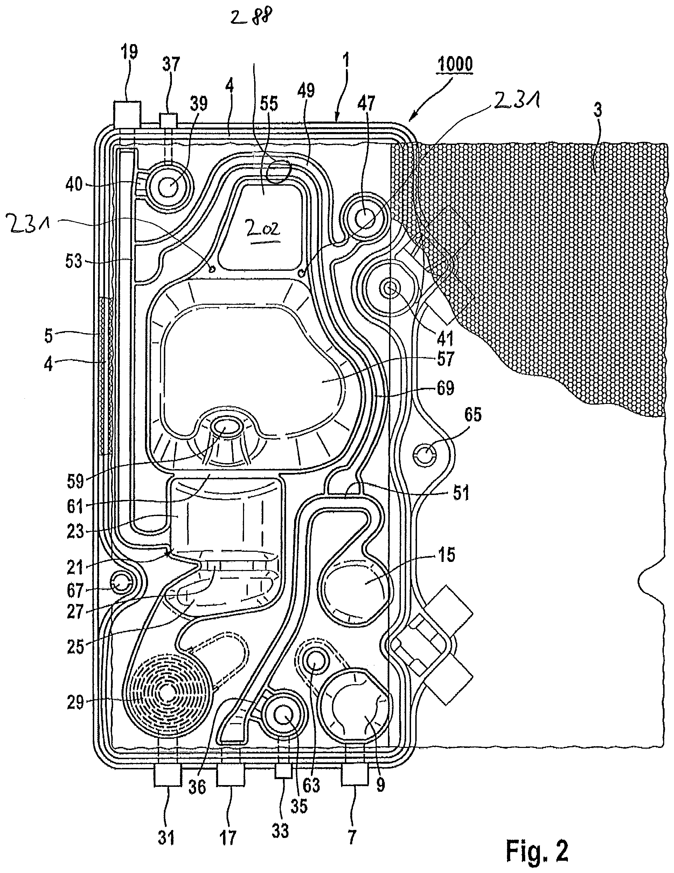

**Fig. 2** zeigt die Kassette 1000 der Fig. 1, wobei die Folie 3 am linken Rand der Kassette 1000 sowie oben und unten zur besseren Illustration zerstörend aufgeschnitten und nach rechts aufgeklappt zu erkennen ist.

Wie in Fig. 2 gezeigt, weist die Folie 3 eine Oberflächenstrukturierung auf.

Fig. 2 zeigt die nach Aufschneiden der Folie 3 detaillierter zu erkennenden Elemente im Inneren der Kassette 1000.

Zur Vermeidung von Wiederholungen wird auf die vorstehend bei der Beschreibung der Fig. 1 erörterten Ausgestaltungen der einzelnen Elemente verwiesen.

Gut zu erkennen ist hierbei, dass die Kassette 1000 einen Dichtsteg 69 aufweist. Der Dichtsteg 69 kann zum Beispiel zum Ausgestalten des Prädilutions-Zugabeventils 51 eingesetzt werden.

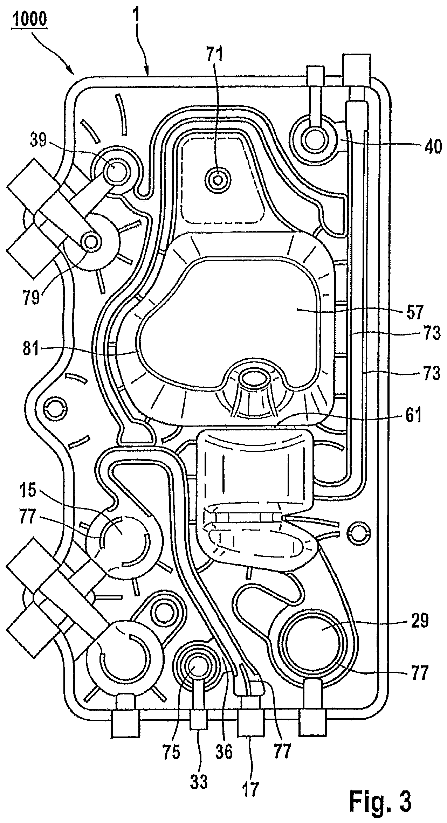

**Fig. 3** zeigt die Kassette 1000 von ihrer Rückseite. Ist die Kassette 1000 an die Blutbehandlungsvorrichtung angekoppelt, so wird ein Betrachter beim Öffnen einer Tür der Blutbehandlungsvorrichtung zum Entnehmen der Kassette 1000 auf diese Rückseite blicken.

Die Kassette 1000 weist einen Single-Needle-Luft-Konnektor 71 auf. Es kann vorgesehen sein, geräteseitig und/oder blutseitig ein Abstützgitter (nicht gezeigt) der Single-Needle-Sterilmembran 55 am Single-Needle-Luft-Konnektor 71 anzuordnen.

Die Kassette 1000 weist mehrere Stützstege auf. Die Stützstege weisen von einander verschiedene Höhen, bezogen beispielsweise auf die Ebene der Folie 3, auf. Die Stützstege erheben sich in der dem Betrachter in Fig. 3 zugewandten Seite der Kassette 1000, also aus der Zeichenebene der Fig. 3 heraus.

Die Kassette 1000 weist Stützstege 73 mit einer Höhe von 5 mm, Stützstege 75 mit einer Höhe von 8 mm, Stützstege 77 mit einer Höhe von 13 mm, Stützstege 79 mit einer Höhe von 24 mm und Stützstege 81 mit einer Höhe von 31 mm auf. Die vorstehenden und andere Zahlenwerte sind natürlich rein exemplarisch zu verstehen.

Die Stützstege können dazu dienen, die Kassette im angekoppelten Zustand an eine Blutbehandlungsvorrichtung gegen einen Deckel einer Aufnahmeeinrichtung der Blutbehandlungsvorrichtung zum Aufnehmen der Kassette abzustützen. Beispielhafte Ausführungsformen einer derartigen Ankopplung der Kassette an die Blutbehandlungsvorrichtung sind in der Patentanmeldung 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, angegeben.

In Fig. 3 ist die Kassette 1000 so gezeigt, wie der Anwender/Betrachter nach ihrem Ankoppeln an das Maschineninterface auf sie blickt. Die Neigung der Kassette 1000 zur Maschine ist "nach hinten geneigt" ausgeführt, so dass die obere Kante weiter vom Anwender/Betrachter entfernt ist als die untere Kante.

Die nach oben zeigenden Flächen der venösen Blutkammer 21 und der Single-Needle-Kammer 57 weisen dementsprechend eine Neigung derart auf, dass Luftblasen trotz der Neigung der Kassette 1000 noch zuverlässig an der Innenseite aufsteigen können. Grundsätzlich ist alternativ natürlich auch ein Kassettendesign möglich, das keine Neigung der Kassette vorsieht.

Die folgenden Figuren zeigen Abschnitte einer erfindungsgemäßen Kassette 1000, welche in allen Merkmalen mit jener Kassette 1000 der Fig. 1 bis 3 übereinstimmen kann, aber nicht muss, soweit sie nicht in den im Folgenden erläuterten Ausführungsformen hiervon abweicht. Jedenfalls kann die erfindungsgemäße Kassette 1000 der folgenden Figuren beliebige Merkmale der in Fig. 1 bis Fig. 3 gezeigten Kassette 1000 aufweisen, sofern die jeweilige Merkmalskombination vom Fachmann nicht als technisch unmöglich erkannt wird.

**Fig. 4** zeigt perspektivisch einen Ausschnitt eines Hartteils 1 einer Kassette 1000, beispielsweise jener der vorangegangenen Figuren. Dieses hat Kanäle 201, eine Kammer 202, umlaufende ebene Kanalrandstege 204, an welchem die Folie 3 mit dem Hartteil 1 verklebt oder verschweißt ist. Dargestellt ist ferner ein gedellt ausgeführter Abschnitt 226 des Hartteils 1, welcher sich in die Kanalrandstege 204 fortsetzt oder diese unterbricht. Die Folie 3 ist nicht mit dem darunter liegenden Abschnitt 226 des Hartteils 1 verklebt oder verschweißt, was die oben beschriebene Ventilwirkung ermöglicht. Der Abschnitt 226 wird hierin auch als Foliendichtsitz-Steg 226 bezeichnet. Anstelle der gedellten Ausgestaltung, wie sie in Fig. 4 gezeigt ist, könnte der Abschnitt 226 auch eben oder gerade verlaufen.

Fig. 4 zeigt ferner zwei Fixierpins 231, mittels welcher ein Federelement 227a oder 227b, wie es in den Fig. 5a oder 5b gezeigt ist, mit dem Hartteil 1 verbunden werden kann.

Auflagehöcker 233, welche im Bereich der Fixierpins 231 vorgesehen sind, sind weiter unter beschrieben.

Die **Fig. 5a** und **5b** zeigen perspektivisch ein Federelement 227a bzw. 227b der Kassette 1000, hierin auch als Disposable-Federelement bezeichnet. Das Federelement 227a ist rein exemplarisch U-förmig oder im Wesentlichen U-förmig und mit wenigstens einem Federbügel 228 und wenigstens einem Federarm oder wenigstens einem Kragarm 228a ausgestaltet. Es dient, ausschließlich oder vornehmlich dazu, in einem vorderen Bereich seines Federbügels 228 eine Anzahl an randständigen Höckern 229a und eine Anzahl an mittig angeordneten
Höckern 229m mit optional zwischen diesen angeordneten Drainagenuten 230 in Stellung zu bringen oder zu positionieren und in einer Richtung senkrecht zur Folie 3 elastisch nachgiebig zu lagern.

Um die Funktion der Ausrichtung, Lagerung und Federung der Höcker 229a, 229b zu erfüllen, ist das Federelement 227a, 227b vorzugsweise aus demselben oder einem ähnlichen Werkstoff wie das Hartteil 1 hergestellt (beispielsweise aus Thermoplast, vorzugsweise mit einem Biege-E-Modul von zirka 800 bis 2000 N/mm²).

Das Federelement 227a, 227b kann aufgrund seiner günstigen Formgebung vorteilhaft extrem preisgünstig in Auf-Zu-Vielfach-Spritzgießwerkzeugen hergestellt werden.

Fig. 5b zeigt eine weitere exemplarische erfindungsgemäße Ausführungsform des Federelements 227b, welches ein integriertes Hydrophobmembran-Abstützgitter 280 aufweist. Anstelle des Hydrophobmembran-Abstützgitters 280 - oder ergänzend hierzu - können Komponenten wie Koagelfängerscheiben, Rückschlagventile usw. im oder am Federelement 227b vorgesehen sein. Auf diese Weise kann die Funktion eines Federelements in ein ohnehin benötigtes Bauteil, wie die vorgenannten, integriert werden, was die Mehrkosten zum Erzielen der Federelement-Funktion vorteilhaft reduziert.

Positioniert und fixiert wird das Federelement 227a, 227b im Beispiel der Fig. 5a, 5b indem seine Fixierbohrungen 232 auf die Fixierpins 231 des Hartteils 1 der Kassette 1000 aufgeschoben, aufgeclippt und/oder thermisch vernietet werden.

Optional vorgesehene Auflagehöcker 233 des Hartteils 1, siehe Fig. 4, und Auflagerippen 234, welche optional auf wenigstens einem der Federarme 228a des Federelements 227a, 227b und vorzugsweise an der Unterseite des Federarms 228a vorgesehen sind, garantieren in bestimmten, exemplarischen Ausführungsformen der vorliegenden Erfindung zusammen mit den Fixierpins 231, welche optional auf Seiten beider Federarme 228a vorgesehen sind, eine spielfreie DreipunktAuflage des Federelements 227a, 227b in der Kammer 202 der Kassette 1000. In einigen, erfindungsgemäßen Ausführungsformen der vorliegenden Erfindung begrenzen die Auflagehöcker 233 die Höhe oder den Winkel, um welche(n) das Federelement 227a, 227b in einem vorderen Bereich hiervon mit den Fixierpins 231 als Drehpunkte vom Hartteil 1 oder von den Kanalrandstegen 204 abheben kann.

Jenseits der Auflage-Rippen 234 - d. h. in Richtung vorderer Abschnitt oder Federbügel 228 des Federelements 227a, 227b weist letzteres keine weiteren Auflagerippen 234 mehr auf.

Aufgrund seiner schlanken Formgebung und seiner sicheren Beabstandung zum Boden und zu den Wänden der Kammer 202 kann das Federelement 227a, 227b frei und elastisch federn.

Im beispielhaften Ausführungsbeispiel der Fig. 5b ist das Federelement 227b durch einen oder mehrere Spalte 235 mechanisch vom Hydrophobmembran-Abstützgitter 280 entkoppelt. Die Funktion des Hydrophobmembran-Abstützgitters 280 bleibt somit vollkommen unberührt von der Funktion und Bewegung des Federelements 227b.

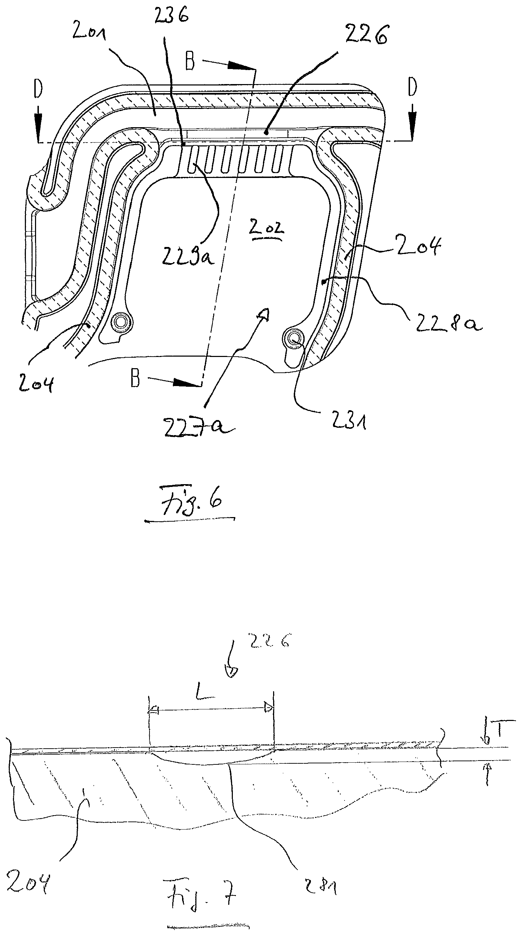

**Fig. 6** zeigt eine Draufsicht auf den in der Fig. 4 dargestellten Ausschnitt einer Kassette 1000 mit eingebautem Federelement 227a der Fig. 5a und mit transparent dargestellter und daher nicht zu erkennender Folie 3, welche an Kanalrandstegen 204 verklebt oder verschweißt ist. Die verklebten oder verschweißten Kanalrandstege 204 haben eine Höhe, auf welcher die geradegezogene Folie 3 spannungsfrei anliegen kann. Kanalrandstege 204 reichen somit an die Folie 3 heran und stünden mit dieser auch ohne Verklebung oder Verschweißung im Wesentlichen in Kontakt.

Der Abschnitt 226 umfasst den Folienventil-Dichtsitz-Steg als Ventilgrund eines als Phantomventil ausgestalteten Ventils oder Folienventils 288, an welchem die Folie 3 nicht mit dem unter ihr liegenden Abschnitt des Hartteils 1 verklebt oder verschweißt ist (bzw. an welchem der über der Folie 3 angeordnete Abschnitt des Hartteils 1 nicht mit der Folie 3 verklebt oder verschweißt ist).

Der Abschnitt 226 kann eben (d. h. mit gerade verlaufender Oberkante) oder gedellt verlaufen (d. h. mit konkaver oder konvexer, jedenfalls aber gekrümmter Oberkante). In der rein exemplarischen, gedellten Ausführung, welche in Fig. 6 gezeigt ist, nimmt die Tiefe der Delle, welche in **Fig. 7** erläuternd in Vergrößerung gezeigt ist, von der geraden Oberkante des Hartteils 1 ausgehend zunächst zu, um nach Erreichen eines tiefstgelegenen Punktes wieder abzunehmen.

Im dargestellten, exemplarischen Fall der Fig. 6 weist der gedellte Abschnitt 226 eine besonders geringe Dellentiefe T bei besonders hoher Dellenbreite und Dichtsitzsteg-Länge L auf, siehe auch Fig. 7 zum besseren Verständnis der vorgenannten Dimensionen, wobei sich die nicht darstellbare Dellenbreite in die Zeichenebene hinein erstreckt und das Bezugszeichen 281 den Dellengrund bezeichnet.

In Verbindung mit dem geringen Spalt 235 zwischen dem Federelement 227a, welche vorzugsweise ca. dem 2- bis 6-fachen der Dicke oder Stärke der Folie 3 entspricht, und aufgrund der hohen Anzahl an Höckern 229a auf dem Federelement 227a ergibt sich bei geringer Dellentiefe T von ca. dem 1- bis 3- fachen der Folienstärke vorteilhaft ein großer strömungstechnischer Verbindungsquerschnitt zwischen dem Kanal 201 und der Kammer 202. Der Verbindungsquerschnitt wird mit einem Folien-Hub von nur dem zirka 2- bis 4-fachen der Folienstärke beim Schließen der Maschinentür verschlossen.

Neben der großen Dimensionierung des Strömungsquerschnittes ist bei dieser elastischen Anordnung der Höcker 229a, 229m ein ungewolltes Schließen des Ventils 288 im Bereich des Abschnitts 226 während der Herstellung der Kassette 1000 und bis zum Abschluss der Gassterilisation besonders wirksam verhindert. Dies liegt auch daran, dass das erfindungsgemäß ausgestaltete Federelement 227a, 227b aufgrund seiner Ausgestaltung und aufgrund seiner sicher ausgestalteten Wegbegrenzung am Boden der Kammer 202 biegetechnisch überlastungssicher ausgelegt ist und die Folie 3 immer sicher vom Dellengrund 281 abhebt, auch wenn die Folie 3 bereits eine Vorschädigung in Form von Dellen aufweisen sollte.

Aufgrund der Auflagerippen 234, der frei biegenden Feder- oder Kragarme 228a sowie der Höcker 229a, 229m mit dazwischen liegenden Drainagenuten 230 kann das Federelement 227a, 227b nur an wenigen Stellen, deren Gesamtfläche nur wenige Quadratmillimeter (mm²) betragen, in direkten Kontakt mit dem Hartteil 1 und mit der Folie 3 stehen. Auf diese Weise wird vorteilhaft die sichere Gassterilisation aller relevanten Oberflächen gewährleistet.

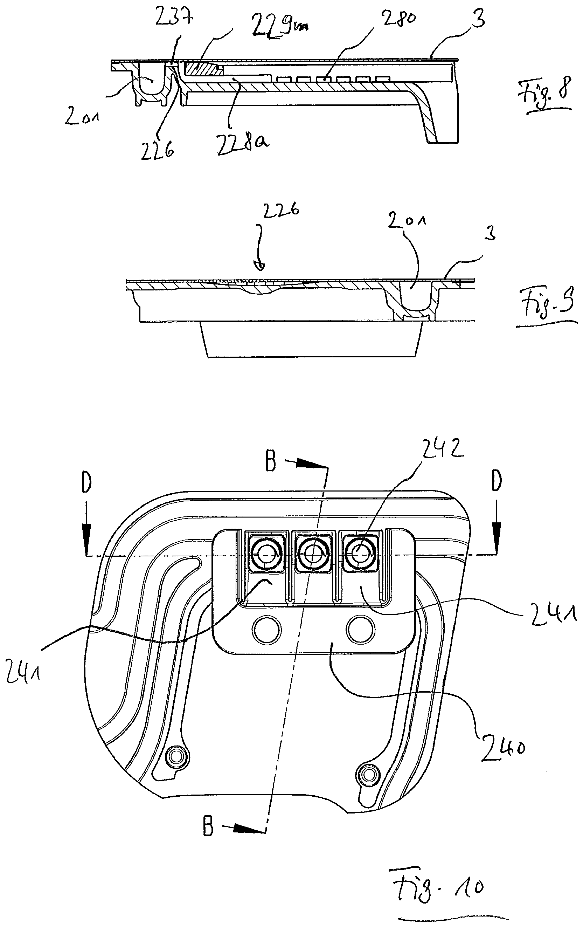

**Fig. 8** und **Fig. 9** zeigen Schnitte durch die noch nicht aufgerüstete (d. h. noch nicht mit der Blutbehandlungsvorrichtung 5000 verbundenen) Kassette 1000 der Fig. 6 als Schnitte entlang der Linien B-B (Fig. 8) bzw. D-D (Fig. 9), jeweils bei horizontal liegender Folie 3.

In dieser Ausgestaltung ist der Abschnitt 226 bzw. der Folienventil-Dichtsitz-Steg oder der Ventilgrund gedellt und weist Höcker 229m auf, welche die Ebene der Folie 3 nicht überragen.

In Fig. 8 bezeichnet das Bezugszeichen 237 zwei Ansichten des durch den Höhenunterschied zwischen Dellengrund 281 und Folie 3 freigehaltenen Strömungsquerschnitts.

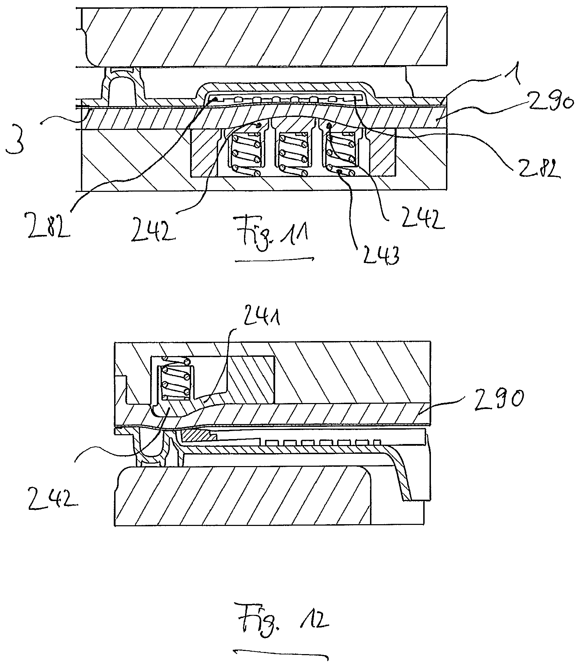

Die randseitigen Höcker 229a und Ecken 282 (siehe Fig. 11) des Federelements 227a sind in der Ausführungsform der Fig. 8 und 9 bewusst flacher oder niedriger gestaltet als die mittleren Höcker 229m (siehe Fig. 11). Die Höcker 229a und 229m bilden bezogen auf Richtung auf den Ventilgrund zu eine leicht gewölbte Silhouette von zirka dem 1- bis 4- fachen der Folienstärke (siehe Fig. 11). Auf diese Weise wird sichergestellt, dass die Höcker 229a, 229m auf dem Federelement 227a, 227b in der Lage sind, die Folie 3 einerseits ausreichend weit vom Ventilgrund abzuheben ohne andererseits dabei durch ihre seitlichen Verklebungs- oder Schweißnähte zu stark behindert zu werden. Die begrenzt steife Folie 3 wird also derart abgehoben, dass sie sich unter der Kraft des Federelements 227a, 227b zu einer nach außen (bezogen auf die Kassette 1000) weisenden Delle umformt. Auf diese Weise gibt sie den gewünschten Strömungsquerschnitt über dem abgeflachten Ventilgrund oder Dichtsitz-Steg 226 frei. Die Ausführungsform mit abgeflachtem Ventilgrund oder Dichtsitz-Steg 226 und elastisch auf dem Federelement 227a, 227b gelagerten (und daher nachgiebigen), gewölbten (d. h. mit unterschiedlicher Höhe, wobei die höchsten Höcker 229m jene in der Mitte sind) angeordneten Höckern 229a, 229m ist besonders vorteilhaft, da sie keine Einbau-Toleranzprobleme in beide Raumrichtungen der Folienebene zwischen Kassette 1000 und Blutbehandlungsvorrichtung 5000 (siehe Fig. 11) verursacht.

**Fig. 10** bis **Fig. 12** zeigen eine Ausführungsform einer in die Blutbehandlungsvorrichtung 5000 eingelegten und von dieser verpressten Kassette 1000, also im aufgerüsteten Zustand, in Draufsicht (Fig. 10) und in Schnittansichten entlang der Linie D-D der Fig. 10 (in Fig. 11) bzw. B-B (in Fig. 12), jeweils mit horizontal ausgerichteter Folienebene.

In Fig. 10 ist von der Blutbehandlungsvorrichtung 5000 nur ein Anpressstempel 240 als Teil einer Aktor-Sensor-Platte 290 zu sehen. Der Anpressstempel 240 ist ein Bespiel eines Aktuators. In dieser beispielhaften erfindungsgemäßen Ausführungsform ist der Anpressstempel 240 in Lamellenstempel 242 (Teil-Aktuatoren) aufgeteilt, siehe die Fig. 11 und 12, die über Biegegelenke 241 an den Grundkörper des Anpressstempels 240 angebunden sind.

Hierbei ergibt sich ein Einbau-Toleranz-Ausgleich zwischen Kassette 1000 und Blutbehandlungsvorrichtung 5000. Ferner verteilt sich die Ventil-Dichtpreßkraft im Gebrauch abnützungsresistent und gleichmäßig auf den gesamten gedellten Ventilgrund. Zur relaxationsfreien Krafterzeugung sind hier beispielhaft Spiralfedern 243 aus Federstahl vorgesehen, die sich in einfacher Weise bei der Montage des Anpressstempels 240 in eine Tasche der Aktor-Sensor-Platte 290 zwischenfügen und vorspannen lassen. Der komplette Anpressstempel 240 kann preiswert aus unverstärkten oder faserverstärkten Thermoplasten in Auf-Zu-Spritzgießwerkzeugen hergestellt werden.

Es sei darauf hingewiesen, dass der Ventilgrund oder der Ventil-Dichtsitz-Steg des Abschnitts 226 keineswegs gedellt sein muss. Auch ein gerader Verlauf des Ventilgrunds ist von der vorliegenden Erfindung umfasst.

**Bezugszeichenliste**

| **Bezugszeichen** | **Beschreibung** |
|---|---|
| 1000 | Kassette |
| 1 | Hartteil |
| 3 | Folie |
| 4 | Dichtsteg |
| 5 | umlaufende Schweißnaht |
| 9 | arterielle Druckmesskammer |
| 11 | Konnektor für Blutaustritt aus Kassette 1000 |
| 13 | Konnektor für Bluteintritt in Kassette 1000 |
| 15 | Kammer mit arterieller Post-Pumpe- bzw. Präfilter-Druckmessstelle |
| 17 | arterielle Filterleitung |
| 19 | venöse Filterleitung |
| 21 | venöse Blutkammer |
| 23 | oberer Raum der venösen Blutkammer 21 |
| 25 | unterer Raum der venösen Blutkammer 21 |
| 27 | Querschnittsverjüngung des Hartteils 1 |
| 29 | Gerinnselfänger |
| 31 | venöser Patientenanschluss |
| 33 | arterielle Heparin-Zugabestelle |
| 35 | Rückschlagventil der arteriellen Heparin-Zugabestelle 33 |
| 36 | arterielles Heparin-Zugabeventil (Phantomventil) |
| 37 | venöse Heparin-Zugabestelle |
| 39 | Rückschlagventil der venösen Heparin-Zugabestelle 37 |
| 40 | venöses Heparin-Zugabeventil (Phantomventil) |
| 41 | Substituat-Zugabestelle |
| 43 | Konnektor für Substituataustritt aus der Kassette 1000 |
| 45 | Konnektor für Substituateintritt in die Kassette 1000 |
| 47 | Rückschlagventil für Substituatzugabe |
| 49 | Substituatleitung |
| 51 | Prädilutions-Zugabeventil (Phantomventil) |
| 53 | Postdilutions-Zugabeventil (Phantomventil) |
| 55 | Single-Needle-Sterilmembran |
| 57 | Single-Needle-Kammer |
| 59 | Blutschwallumleitungselement |
| 61 | Single-Needle-Blutventil (Phantomventil) |
| 63 | Absaugstelle für Vakuumankopplung |
| 65 | primäres Ausrichtungszentrum |
| 67 | sekundäre Ausrichtungsstelle |
| 69 | Dichtsteg |
| 71 | Single-Needle-Luft-Konnektor |
| 73 | Stützstege mit 5 mm Höhe |
| 75 | Stützstege mit 8 mm Höhe |
| 77 | Stützstege mit 13 mm Höhe |
| 79 | Stützstege mit 24 mm Höhe |
| 81 | Stützstege mit 31 mm Höhe |
| 201 | Kanal |
| 202 | Kammer |
| 204 | umlaufende ebene Kanalrandstege |
| 226 | Abschnitt oder Foliendichtsitz-Steg oder Ventilsitz oder Ventilgrund |
| 227a, 227b | Federelement oder Disposable-Federelement |
| 228 | Federbügel |
| 228a | Federarmen oder Kragarme |
| 229a, 229m | Höcker |
| 230 | Drainagenuten |
| 231 | Fixierpin |
| 232 | Fixierbohrungen |
| 233 | Auflagehöcker |
| 234 | Auflagerippen |
| 235 | Spalt |
| 237 | Höhenunterschied oder Strömungsquerschnitt |
| 240 | Aktuator oder Anpressstempel |
| 241 | Aktuator, Biegegelenke |
| 242 | Teil-Aktuator oder Teil-Anpressstempel |
| 243 | Spiralfeder |
| 280 | Hydrophobmembran-Abstützgitter |
| 281 | Dellengrund |
| 282 | Ecken |
| 288 | Phantom- oder Folienventil |
| 290 | Aktor-Sensor-Platte |
| 5000 | Blutbehandlungsvorrichtung |

## Patentansprüche

1. Blutbehandlungskassette (1000) mit einem als Hartteil (1) ausgestalteten Kassettenkörper und einer Folie (3), wobei die Folie (3) mit dem Hartteil (1) verbunden ist und das Hartteil (1) zumindest abschnittsweise abdeckt,
wobei das Hartteil (1) wenigstens einen Ventilgrund (226) eines Ventils (288) aufweist, wobei das Ventil (288) ausgestaltet ist, um neben einer ersten, geöffneten Stellung des Ventils (288), in welcher der Ventilgrund (226) und ein über diesem angeordneter Abschnitt der Folie (3) einander nicht berühren, bei Aufbringen einer Kraft auf einen Abschnitt der Folie (3) eine zweite, geschlossene Stellung des Ventils (288) einzunehmen, in welcher der Ventilgrund (226) und der Abschnitt der Folie (3) einander berühren,
wobei die Blutbehandlungskassette (1000) wenigstens einen federnd oder elastisch am Hartteil (1) abgestützten und/oder mit diesem integral hergestellten Abstandhalter aufweist,
wobei der Abstandhalter angeordnet ist, um in der ersten Stellung Spannung, Kraft oder Druck auf den über dem Ventilgrund (226) angeordneten Abschnitt der Folie (3) auszuüben,
wobei der Abstandhalter integrales Element eines mit dem Hartteil (1) verbundenen Federelements (227a; 227b) ist oder hieran angeordnet ist,
wobei auf dem Federelement (227a; 227b) Höcker angeordnet sind, welche von Drainagestrukturen (230) durchzogen sind und/oder wobei das Federelement (227a; 227b) ein Funktionselement, nämlich ein integriertes Hydrophobmembran-Abstützgitter (280), eine Koagelfängerscheibe und/oder ein Rückschlagventil aufweist.

2. Blutbehandlungskassette (1000) nach Anspruch 1, wobei das Ventil (288) ausgestaltet ist, um mittels Druckaufbringung eines Aktuators (240, 241, 242) einer Blutbehandlungsvorrichtung (5000) auf das Ventil (288), zu deren Betrieb die Blutbehandlungskassette (1000) bestimmungsgemäß mit der
Blutbehandlungsvorrichtung (5000) verbunden wird, von der ersten Stellung in die zweite Stellung überführbar zu sein.

3. Blutbehandlungskassette (1000) nach Anspruch 1 oder 2, wobei das Ventil (288) als Folien- oder Phantomventil ausgestaltet ist.

4. Blutbehandlungskassette (1000) nach einem der vorangegangenen Ansprüche, wobei zwischen dem Federelement (227a; 227b) und dem Funktionselement wenigstens ein Spalt (235) oder Spaltabschnitt oder eine Nut vorgesehen ist.

5. Blutbehandlungskassette (1000) nach einem der vorangegangenen Ansprüche, wobei eine Dellentiefe (T) des Ventilgrunds (226) dem 1- bis 3-fachen der Dicke oder Stärke der Folie (3) entspricht oder der
Ventilgrund (226) hinter benachbarten
Kanalrandstegen (204) um das 1- bis 3-fache der Dicke oder Stärke der Folie (3) in Richtung Inneres der Kassette (1000) zurückgesetzt ist.

6. Blutbehandlungskassette (1000) nach einem der vorangegangenen Ansprüche, wobei der Abstandhalter bezogen auf die Richtung auf den Ventilgrund (226) eine gewölbte Oberkante aufweist, deren Wölbung in vorgenannten Richtung um bis zu das 1- bis 4- fache der Dicke oder Stärke der Folie (3) erhebt.

7. Blutbehandlungsvorrichtung (5000) mit einer Blutbehandlungskassette (1000) nach einem der Ansprüche 1 bis 6, mit einer Aktor-Sensor-Platte (290), wobei die Aktor-Sensor-Platte (290) wenigstens einen Aktuator (240) aufweist, welcher wenigstens zwei, vorzugsweise mehr als drei, Teil-Aktuatoren (242) aufweist, welche angeordnet sind, um unabhängig voneinander Kraft auf den Abschnitt der Folie (3) des Ventils auszuüben.

## Claims

1. A blood treatment cassette (1000) comprising a cassette body designed as a hard part (1) and a film (3), the film (3) being connected to the hard part (1) and covering the hard part (1) at least in sections,
wherein the hard part (1) comprises at least one valve base (226) of a valve (288), the valve (288) being designed to take, in addition to a first, open position of the valve (288), in which the valve base (226) and a portion of the film (3) disposed above it don't touch each other, a second, closed position of the valve (288), in which the valve base (226) and the portion of the film (3) touch each other when a force is applied to a portion of the film (3),
wherein the blood treatment cassette (1000) comprises at least one spacer springly or elastically supported on the hard part (1) and/or produced integrally therewith,
wherein the spacer is arranged to apply tension, force or pressure to the portion of the film (3) disposed above the valve base (226) in the first position.
wherein the spacer is an integral element of a spring element (227a; 227b) connected to the hard part (1) or is arranged at it,
wherein humps are arranged on the spring element (227a; 227b), the humps being traversed by drainage structures (230) and/or wherein the spring element (227a; 227b) comprises a functional element, namely an integrated hydrophobic membrane support grid (280), a blood clot catcher disc and/or a non-return valve.

2. The blood treatment cassette (1000) according to claim 1, wherein the valve (288) is designed to be transferable from the first position to the second position by applying a pressure with an actuator (240, 241, 242) of a blood treatment apparatus (5000) to the valve (288), for the operation of which the blood treatment cassette (1000) is as intended connected to the blood treatment apparatus (5000).

3. The blood treatment cassette (1000) according to claim 1 or 2, wherein the valve (288) is designed as a film valve or as a phantom valve.

4. The blood treatment cassette (1000) according to anyone of the preceding claims, wherein at least a gap (235) or a gap portion or a groove is provided between the spring element (227a; 227b) and the functional element.

5. The blood treatment cassette (1000) according to anyone of the preceding claims, wherein a depression depth (T) of the valve base (226) corresponds to 1 to 3 times the thickness or strength of the film (3), or wherein the valve base (226) is set back behind adjacent channel border bridges (204) up to 1 to 3 times the thickness or strength of the film (3) towards the interior of the cassette (1000).

6. The blood treatment cassette (1000) according to anyone of the preceding claims, wherein the spacer comprises a curved upper edge relative to the direction of the valve base (226), whose curvature can reach up 1 to 4 times the thickness or strength of the film (3) in the aforementioned direction.

7. A blood treatment apparatus (5000) comprising a blood treatment cassette (1000) according to anyone of claims 1 to 6, with an actuator sensor plate (290), wherein the actuator sensor plate (290) comprises at least one actuator (240) having at least two, preferably more than three, partial actuators (242) arranged to exert, independently to each other, a force on the portion of the film (3) of the valve.

## Revendications

1. Une cassette de traitement du sang (1000) comprenant un corps de cassette conçu comme une partie rigide (1) ainsi qu'un film (3), le film (3) étant relié à la partie rigide (1) et recouvrant la partie rigide (1) au moins par sections,
où la partie rigide (1) comprend au moins un fond de vanne (226) d'une vanne (288), la vanne (288) étant conçue pour prendre, en plus d'une première position ouverte de la vanne (288), dans laquelle le fond de vanne (226) et une section du film (3) disposé au-dessus de celui-ci ne se touchent pas,
une seconde position fermée de la vanne (288), dans laquelle le fond de vanne (226) et la section du film (3) se touchent lorsqu'une force est appliquée sur une section du film (3),
où la cassette de traitement du sang (1000) comprend au moins une entretoise soutenue de manière flexible ou élastique sur la partie rigide (1) et/ou réalisée intégralement avec celle-ci,
où l'entretoise est agencée de façon à appliquer une tension, une force ou une pression sur la section du film (3) disposée au-dessus du fond de vanne (226) dans la première position.
où l'entretoise est un élément intégral d'un élément ressort (227a; 227b) relié à la partie rigide (1) ou disposé sur celle-ci,
où, sur l'élément ressort (227a; 227b) sont disposés des renflements traversés par des structures de drainage (230) et/ou où l'élément ressort (227a; 227b) comprend un élément fonctionnel, notamment une grille de support de membrane hydrophobe intégrée (280), un disque collecteur de caillots sanguins et/ou une vanne anti-retour.

2. La cassette de traitement du sang (1000) selon la première revendication, où la vanne (288) est conçue pour être transférable de la première position à la seconde position par application d'une pression au moyen d'un actionneur (240, 241, 242) de l'appareil de traitement du sang (5000) sur la vanne (288), pour le fonctionnement duquel la cassette de traitement du sang (1000) est reliée à l'appareil de traitement du sang (5000), conformément à son usage prévu.

3. La cassette de traitement du sang (1000) selon la revendication 1 ou 2, où la vanne (288) est conçue comme une vanne à membrane ou une vanne fantôme.

4. La cassette de traitement du sang (1000) selon l'une quelconque des revendications précédentes, où au moins une encoche (235) ou une section d'encoche ou une rainure est prévue entre l'élément ressort (227a; 227b) et l'élément fonctionnel.

5. La cassette de traitement du sang (1000) selon l'une quelconque des revendications précédentes, où une profondeur d'enfoncement (T) du fond de vanne (226) correspond à 1 à 3 fois l'épaisseur ou la hauteur du film (3), ou où le fond de vanne (226) est décalé de 1 à 3 fois l'épaisseur ou la hauteur du film (3) vers l'intérieur de la cassette (1000) à l'arrière des bords de traverses de conduits (204) adjacents.

6. La cassette de traitement du sang (1000) selon l'une quelconque des revendications précédentes, où l'entretoise présente un bord supérieur incurvé par rapport à la direction du fond de vanne (226), dont la courbure peut atteindre jusqu'à 1 à 4 fois l'épaisseur ou la hauteur du film (3) dans la direction précitée.

7. Un appareil de traitement du sang (5000) comprenant une cassette de traitement du sang (1000) selon l'une quelconque des revendications 1 à 6, avec une plaque
actionneur-capteur (290), où la plaque
actionneur-capteur (290) comprend au moins un
actionneur (240) ayant au moins deux, de préférence plus de trois, actionneurs partiels (242) agencés de façon à pouvoir exercer, indépendamment les uns des autres, une force sur la section du film (3) de la vanne.
